# EUROPEAN PATENT APPLICATION

(11) **EP 2 527 386 A1**
(43) Date of publication of application: **28.11.2012**
(21) Application number: 11734632.0
(22) Date of filing: 18.01.2011
(51) Int. Cl.: C08G 61/12, C07D 333/32

(54) **REGIOREGULAR POLYTHIOPHENE AND PROCESS FOR PRODUCTION THEREOF**

(30) Priority: 19.01.2010 JP 2010009279
(71) Applicant: Kuraray Co., Ltd., Okayama 710-0801 (JP)
(72) Inventor: KAMADA, Taisuke, Kurashiki-shi Okayama 710-0801 (JP); NAKAI, Shinji, Kurashiki-shi Okayama 710-0801 (JP)
(74) Representative: von Kreisler Selting Werner
(86) International application number: PCT/JP2011/050751
(87) International publication number: WO 2011/090026

(57) **Abstract**

Disclosed is a polythiophenes having high electrical conductivity, excellent solubility, long-tern stability, excellent processability and high regioregularity. Specifically disclosed is regioregular polythiophene represented by chemical formula (1) 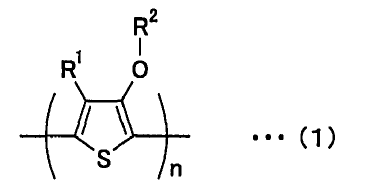 wherein R¹- represents a 4-substituent comprising an alkyl having 1 to 20 carbon atoms; R²-O- represents a 3- substituent comprising an alkyl having 1 to 20 carbon atoms; and n represents such a numeral value that the number average molecular weight of the regioregular polythiophene becomes 200 to 1,000,000), wherein the regioregularity of the Head-Tail repeating structure is at least 98%.

## Description

### Technical Field

The present invention relates to a high electroconductive regioregular polythiophene that is used for electroconductive material and to a process for producing the polythiophene from its raw material.

### Background of the Invention

Polymers which are obtained by polymerizing a compound having a five- or six-membered ring structure such as pyrrole, thiophene, aniline, etc. having an endocyclic or exocyclic hetero atom or by polymerizing a compound having a hydrocarbon series aromatic ring structure, have an electroconductive property. Such polymers are conductive organic materials whose electroconductivity can be appropriately controlled by adjusting the amount of a dopant coexisting together with the polymers and have a good processability. Accordingly, the use of various conductive organic materials for various electrodes, sensors, primary cells, secondary cells, solid electrolytic capacitors, antistatic agents, etc. has been studied.

Among those polymers, it is known that poly (3-methoxy-4-methylthiophene) has a relatively high electroconductivity (Japanese Patent Publication 2005-154481A; M. Feldhues et al., "Polyalkoxythiophenes soluble electrically conducting polymer" Synthetic Metals, 1989, vol. 11, C487-C493). Poly (3-methoxy-4-methylthiophene) obtained in the past had regioregularity, or a ratio of regioregular arrangement of the so-called Head-Tail (head to tail) of the repeating unit, was about 85% at best. Further, it has been known that a polythiophene having a long chain substituent in the 3-position or 4-position of the thiophene have a long-term stability but the substituent exhibits an adverse effect on their electroconductivity because its regioregularity becomes low with the length of the substituent (Richard D. Mc Cullough et al., (Self-Orienting Head-Tail Poly (3-alkylthiophenes): New insights on Structure-Property Relationships in Conducting Polymers), Journal of the American Chemical Society, 1993, vol. 115, p. 4910-4911). In addition, there is a difficulty in film forming (processability) because of its poor solubility, so improvement in regioregularity and solubility has been desired.

It has been known that thiophene derivatives, which has a long chain alkoxy group as 3-substituent instead of a 3-methoxy group of 3-methoxy-4-methylthiophene, can provide a polymer exhibiting a regioregularity of up to 96% and relatively high solubility. Such polymer can be obtained using a chemical oxidative polymerization method in the presence of a transition metal salt such as ferric chloride (Naoyuki Koide et al., Synthesis and Physical Property of regioregular Poly (3-alkoxy-4-methylthiophene)s, Polymer Journal, 2004, vol.36, No. 8, p. 628-633; Japanese Patent Publication 2005-154481A). However, further improvement in regioregularity, electroconductivity and solubility has been desired.

### Summary of the Invention

The present invention is made to solve the above mentioned problems. An object of the present invention is to provide a polythiophenes having high regioregularity, high electroconductivity, excellent solubility, long-term stability, excellent processability, and to provide a process for producing highly pure polythiophenes easily and in good yield from its starting compounds.

A regioregular polythiophene developed to achieve the aforementioned object of the present invention is represented by the following chemical formula (1) 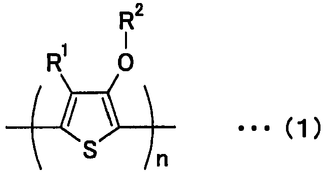 wherein R¹- represents a 4-substituent comprising an alkyl having 1 to 20 carbon atoms; R²-O- represents a 3-substituent comprising an alkyl group having 1 to 20 carbon atoms; and n represents such a numeral value that the number average molecular weight of the regioregular polythiophene becomes 200 to 1,000,000, wherein the regioregularity of the Head-Tail repeating structure is at least 98%.

A raw material composition of the conductive material is comprised of: a polythiophene represented by the following chemical formula (1); and a dopant, 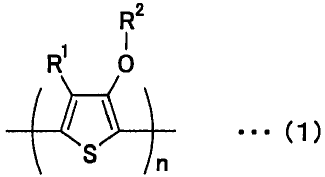 (in formula (1); R¹ - represents a 4-substituent comprising an alkyl having 1 to 20 carbon atoms; R²-O- represents a 3-substituent comprising an alkyl having 1 to 20 carbon atoms; and n represents such a numeral value that the number average molecular weight of the polythiophenes becomes 200 to 1,000,000), wherein the regioregularity of the Head-Tail repeating structure is at least 98%.

A process for producing polythiophene is comprised of a step of: polymerizing a thiophene compound monomer represented by the following chemical formula (2), 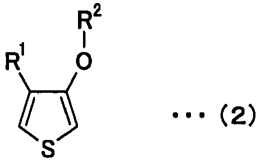 wh erein R¹- represents a 4-substituent comprising an alkyl having 1 to 20 carbon atoms; R²-O- represents a 3-substituent comprising an alkyl having 1 to 20 carbon atoms, using an electrolytic polymerization method or a chemical oxydative polymerization method in the presence of ferric perchlorate to induce the regioregularity of the Head-Tail repeating structure in the polythiophene represented by the following chemical formula (1), 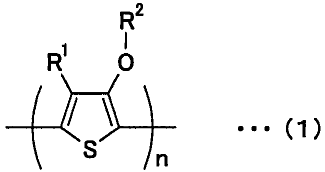 (in formula (1): R¹- and R²-O- are the same as described in the formula (2) shown above, and n represents such a numeral value that the number average molecular weight of the regioregular polythiophene becomes 200 to 1,000,000) wherein the regioregularity of the Head-Tail repeating structure is at least 98%.

A thiophene compound monomer is represented by the following chemical formula (3) 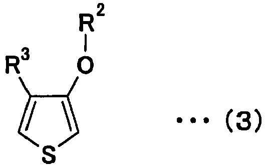 w herein R³- is a 4-substituent comprising an alkyl having 6 to 20 carbon atoms; R²-O- represents a 3-substituent comprising an alkyl having 1 to 20 carbon atoms.

Regioregular polythiophene of the present invention have high regioregularity of 98% or over. And a Head-Tail repeating structure is aligned regioregularly. Because of this high regioregularity, the present polythiophenes has high conductivity, excellent solubility, and long-term stability. A raw material composition of an electroconductive material comprising the polythiophenes and a dopant can be easily prepared as a homogeneous raw material for forming a high quality electroconductive material exhibiting high electroconductivity according to the polythiophenes' high conductivity.

According to a method for producing the polythiophenes of the present invention, the polythiophenes with high regioregularity can be produced easily, in large quantity and high yield, without troublesome steps, using a simple manufacturing equipment.

A thiophene compound monomer of the present invention is a starting compound from which such regioregular polythiophene can be produced easily by only one process.

Hereinafter, preferred embodiments of the present invention will be precisely explained below, but the scope of the present invention should not be limited to the embodiments described here.

The regioregular polythiophene of the present invention is represented by the following chemical formula (1) 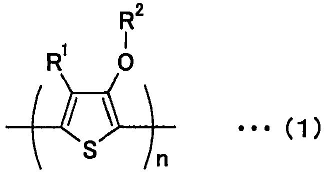 an
d can be a single compound or a mixture of compounds having a repeating unit whose number of repetition is an integer of 2 or more, and that the 2-position of the thiophene ring of the repeating unit and the 5-position of an adjacent thiophene ring are bonded to each other to constitute a Head-Tail repeating structure, the amount of which in the polythiophene is 98 mole % or over, and n represents such a numeral value that the number average molecular weight of the polythiophenes becomes 200 to 1,000,000. In the molecular structure of this polythiophenes, the total mole % of the Head-Head and the Tail-Tail repeating structures, which are formed by bonding of a 2-position (or 5-position) of one thiophene ring with another 2-position (or 5-position) of an adjacent thiophene ring, is less than 2 mole% or an undetectable level when measured using an instrument analysis such as a nuclear magnetic resonance spectrum, etc.

In the formula (1), R¹- represents a 4-substituent comprising a linear, branched and/or cyclic alkyl having 1 to 20 carbon atoms, R²-O- represents a 3- substituent comprising a linear, branched and/or cyclic alkyl having 1 to 20 carbon atoms. These alkyl groups may be an alkyl group represented by an unsubstituted CₘH₂ₘ₊₁ (m=1 to 20). Or such alkyl group may be substituted with a group such as a halogen atom, an alkoxy group having carbon atom of not more than 20 or an aryl group. As the alkyl group in such 3- or 4-substituent, for example, methyl group, ethyl group, n-propyl group, isopropyl group, n-butyl group, isobutyl group, sec-butyl group, tert-butyl group, n-pentyl group, isopentyl group, neopentyl group, tert-pentyl group, n-hexyl group, isohexyl group, 2-ethylhexyl group, n-heptyl group, n-octyl group, n-nonyl group, n-decyl group, n-undecyl group, n-dodecyl group, n-tetradecyl group, n-pentadecyl group, n-hexadecyl group, n-heptadecyl group, n-octadecyl group, n-nonadecyl group, n-icosyl group, etc. can be exemplified.

These regioregular polythiophene can be obtained by a producing method described below.

As a starting material, 3-bromothiophene compound (4) whose 4-position is substituted by 4-substituent comprising an alkyl group (which may be a commercially available one) or one that is synthesized from a raw material such as 3-alkylthiophene compound whose 4-position is substituted with 4-substituent comprising an alkyl and whose 2-, 3- and 5-positions are brominated with bromine, then treated with a base to carry out debromination at 2- and 5-positions, can be used. At first, from this compound (4), thiophene compound monomer (2) can be obtained through step 1 as shown in chemical reaction equation [I] 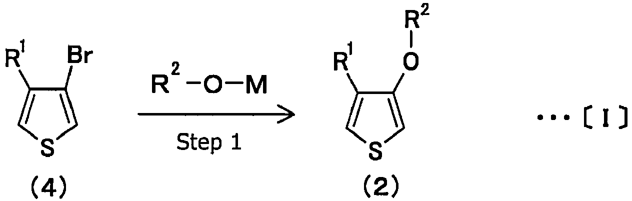 wherein R¹- and R²-O- are the same as described in aforementioned formula (1), and M is a metal.

In the presence of copper reagent and in an inactive solvent, etherification is carried out with a metal alkoxide (5) such as sodium alkoxide to produce thiophene compound monomer (2).

R²-O-M (5)

(in formula (5), R²-O- is the same as described in aforementioned formula (1), and M is a metal.)

The copper reagent which is used in step (1) is not particularly limited so far as it catalyzes and promotes the etherification, but a copper powder, or a copper halide (I) such as copper chloride (I), copper bromide (I), copper iodide (I), etc, can be exemplified. Copper halide is preferably used because it exhibits good etherification reactivity. Above all, copper bromide (I) is much preferably used. The used amount of the copper reagent is not particularly limited, but preferably 0.01 to 1 molar equivalents per 1 mole of 3-bromothiophene compound (4) which is substituted by 4-substituent comprising an alkyl group.

The inactive solvent used in the step (1) is not particularly limited so far as it does not adversely affect the etherification reaction, but an aprotic polar solvent is preferably used. Specifically, an amide series solvent such as N-methyl formamide, N-ethylformamide, N, N-dimethyl formamide, N, N-dimethyl acetamide, N-methylpyrrolidinone, 1, 3-dimethyl-2-imidazolidinone, etc., and a sulfoxide series solvent such as dimethyl sulfoxide, methylethyl sulfoxide, diethyl sulfoxide, etc., can be exemplified. These solvents can be used alone or in combination thereof. The used amount of the inactive solvent is not particularly limited, but preferably 0.1 to 100 parts by mass per 1 part by mass of 3-bromo thiophene compound (4) which is substituted by 4-substituent comprising an alkyl group, is used.

A reaction temperature at an etherification reaction in step 1 is not particularly limited, but the temperature is preferably at 80 to 200°C. When the reaction temperature is set at not more than 80°C, the etherification reaction rate may become extremely slow. The reaction temperature is preferably higher than 100°C, still more preferably higher than 130°C. On the other hand, in the case where the chemical reaction temperature exceeds 200°C, decomposition of the reaction product may occur, so that the reaction temperature is more preferably not higher than 180°C.

After the etherification reaction in step 1, if necessary, isolation and purification of the thiophene compound monomer (2), which is the crude product, may be carried out. Specifically, an organic solvent such as toluene, ethyl acetate, methylene chloride, etc. and water are added into the reaction mixture of the etherification reaction, then extraction is performed using a separating funnel, and then an organic phase is separated from a water phase. Then the organic phase is dried under anhydrous sodium sulfate, then concentrated. As the purification of the obtained crude product, for example, recrystalization, distillation, silica gel column chromatography etc. can be exemplified. According to these proceedings, more highly pure thiophene compound monomer (2) can be obtained.

The thiophene compound monomer represented by formula (3) shown below is more preferably used than thiophene compound monomer represented by (2) in step 1. 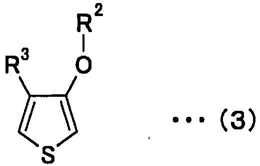 (in formula (3), R³- represents a 4-substituent comprising a linear, branched and/or cyclic alkyl having 6 to 20 carbon atoms, R²-O- represents a 3-substituent comprising a linear, branched and/or cyclic alkyl having 1 to 20 carbon atoms as previously described). R³- may be an alkyl group represented by CₙH₂ₙ₊₁ (n=6 to 20), or such alkyl group may be substituted with a halogen atom, an alkoxy group, an aryl group, etc. as previously shown. As such alkyl group in R³-, for example, n-hexyl group, isohexyl group, 2-ethylhexyl group, n-heptyl group, n-octyl group, n-nonyl group, n-decyl group, n-undecyl group, n-dodecyl group, n-tetradecyl group, n-pentadecyl group, n-hexadecyl group, n-heptadecyl group, n-octadecyl group, n-nonadecyl group, n-icosyl group, etc. can be exemplified.

Next, as shown in chemical reaction equation [II] which has step 2 shown below, thiophene compound monomer (2) is oxidized during polymerization reaction, and regioregular polythiophene, a polymer represented by formula (1), can be obtained through step (2). 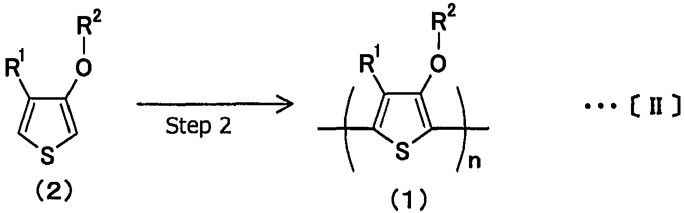 (in chemical reaction equation [II], R¹-, R²-O- and n are the same as previously shown in formula (1))

Regioregular polythiophene (1) is represented as a neutral state, as shown in chemical reaction equation [II]. However when it actually offers conductive properties, it becomes a salt that comprises: a cation comprising the regioregular polythiophene, a polymer shown by positively charged formula (1); and a dopant which serves as a counter anion.

Polymerization reaction in step 2 is carried out preferably using a chemical oxidation polymerization. In the case of chemical oxidation polymerization, a method, in which thiophene compound monomer (2) is oxidized by dehydrogenation using ferric perchlorate, can be preferably adopted, to obtain regioregular polythiophene (1) as a polymer. The used amount of ferric perchlorate per thiophene compound monomer (2) is preferably in the range of 400 to 1,000 mol%. In this instance, obtained is a composition ingredient comprising a salt of a positively charged regioregular polythiophene (1) and a perchlorate anion. In the chemical oxidation polymerization, as a solvent used for a reaction liquid, for example, chloroform, acetonitrile, methanol, ethanol, benzene, toluene, tetrahydrofuran, etc. can be exemplified.

The polymerization reaction in step 2 may be carried out through electrolytic polymerization, as another embodiment. In the case of electrolytic polymerization, a method to obtain the regioregular polythiophene, which comprises steps of: preparing an electrolyte solution into which thiophene compound monomer (2) is dissolved; and applying voltage on electrodes which are arranged via the electrolyte solution to obtain an anodically oxidized regioregular polythiophene on an anode, is preferably adopted. In the electrolytic polymerization, a voltage of 0.3V to 2.0V is preferably applied to the anode with respect to (Ag/Ag⁺) reference electrode. In this electrolytic polymerization, for example, nitromethane, acetonitrile, propylene carbonate, nitrobenzene, cyanobenzene, o-dichlorobenzene, dimethylsulfoxide, γ-butyrolactone, methylene chloride, etc. can be exemplified as the solvent used for the electrolyte solution. As a supporting electrolyte which is added into the electrolyte solution, a combination of, ion of alkali metals such as lithium ion, potassium ion, sodium ion, etc. or a cation such as quaternary ammonium ion; and an anion such as perchlorate ion, boron tetrafluoride ion, phosphorus hexafluoride ion, halogen atom ion, arsenic hexafluoride ion, antimony hexafluoride ion, sulfate ion, hydrogen sulfate ion, etc., can be exemplified. In addition, as an electrolyte solution, an ionic liquid containing an alkyl imidazolium salt, an alkyl pyridinium salt, etc. can be used. As an electrode material, platinum, gold, nickel, indium tin oxide (ITO), etc., can be used. In this instance, obtained is a composition ingredient comprising a salt of such anion and a positively charged regioregular polythiophene (1).

The raw material composition of the conductive material of the present invention comprises polythiophenes represented by formula (1) and a dopant. It is preferable that polythiophenes (1) serves as a cation component and the dopant serves as an anion component or a counter ion of polythiophenes (1).

The dopant serves electrostatically as a counter anion with respect to the regioregular polythiophene having a constitutional unit represented by positively charged formula (1). Specifically, halide anions of Group 5B elements such as PF₆⁻, SbF₆⁻, AsF₆⁻, etc.; halide anions of Group 3B element such as BF₄-, etc.; halogen anions such as I- (or I₃⁻), Br⁻, CI⁻, etc.; a halogen acid anion such as CIO₄⁻, etc.; metal halide anion such as AICI₄⁻, FeCI₄⁻, SnCI₅⁻ , etc.; nitrate anion represented by NO₃⁻, sulfate anion represented by SO₄²⁻; organic sulfonate anion such as p-toluene sulfonate anion, naphthalensulfonate anion, CH₃SO₃⁻, CF₃SO₃⁻ etc.; carboxylic acid anion such as CF₃COO⁻, C₆H₅COO⁻, etc.; and a modified polymer having these anion species on a main chain or a side chain, etc., are exemplified. The dopant of such anion can be used alone or in combination of 2 or more dopants. A method to add a dopant or an anion is not particularly limited, but, for example, a desired anion can be added after the polymerization step. In a case where polymerization is carried out using chemical oxidative polymerization, an anion derived from oxidant or ferric perchlorate can be used as it is as a dopant. When polymerization is performed using electrolytic polymerization, an anion which is derived from electrolyte can be used as it is as a dopant.

The polythiophenes which is a polymer represented by formula (1), in particular, one that has a long chain length at 3-position, 4-position of the ring has far high regioregularity, high solubility in organic solvent and excellent processability when compared with poly (3-methoxy-4-methylthiophene) and can be preferably used for electrical conducting material.

Examples of the synthesis of the thiophene compound monomer, of the production of the regioregular polythiophene using the monomer and a raw-material composition for a conductive material containing the regioregular polythiophene of the present invention, will be further specifically described below.

### (Example)

In a 25ml three-necked flask, a reaction container, with a dropping funnel and thermometer, 3.19g (59.0mmol) of sodium methoxide, 2.43g (9.83mmol) of 3-bromo-4-hexylthiophene represented by formula (4a), 353mg (2.46mmol) of copper bromide (I) and 6.0ml of N-methylpyrrolidinone were placed, then the reaction container was heated up to 140°C. After the reaction was completed, 200ml of toluene and 200ml of water were added to separate an organic phase from a water phase. The organic phase was washed twice each with 200ml of water, dried under anhydrous sodium sulfate and then concentrated to obtain a crude product. This crude product was purified using a silica gel column chromatography (developing solvent: ethyl acetate and n-hexane), obtaining 3-hexyl-4-methoxy thiophene (1.70g, 8.55mmol, yield 87.0%) represented by formula (2a) shown below.

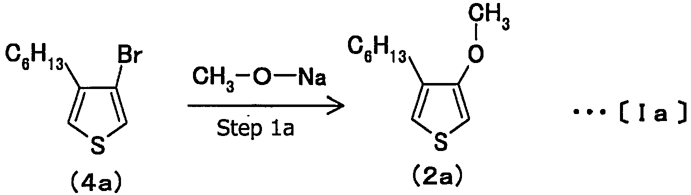

Shown below are its ¹H-NMR spectrum data which support the structure represented by formula (2a).

¹H-NMR (270MHz, CDCl₃, TMS) δ: 6.81 (d-t, 1H, J = 3.5Hz, 1.1Hz), 6.61 (d, 1H, 3.5Hz), 3.81 (s, 3H), 2.46 (d-t, 2H, J = 7.6, 1.1Hz), 1.59 (m, 2H), 1.31 (m, 6H), 0.88 (m, 3H)

Into an electrolytic cell, in which an ITO coated glass plate (surface resistance value: 10 Ω/□) as an anode, a platinum wire as a cathode and a silver/silver perchlorate (10mM acetonitrile solution) as a reference electrode were arranged, 10mL of 10mM tetrabutyl ammonium perchlorate/acetonitrile solution was transferred and 198mg (1.00mmol) of 3-hexyl-4-methoxythiophene represented by formula (2a) was dissolved, then the air was displaced with nitrogen gas. Each electrode in the electrolytic cell was connected to a potentiostat/galvanostat HAB-151 (produced by Hokuto Denko Corporation), then a constant voltage of 1.1V was applied in a potentiostat mode to perform electrolytic polymerization. A black-colored polymer film, which was the regioregular polythiophene, was produced on the anode.

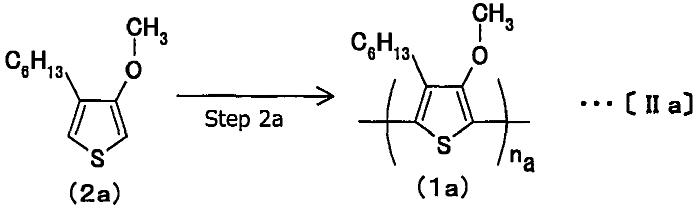

The film produced here was washed with dehydrated acetonitrile, and dried. Its electroconductivity was measured to be 1.1 S/cm using a four-prove method. It was found that this polymer film was a good electroconducting material. A neutral-state polymer obtained from the produced film by applying constant voltage of -0.5V was soluble in an organic solvent such as chloroform, methylene chloride, tetrahydrofuran, etc. It was also found that this polymer has an excellent processability.

The number average molecular weight of this polymer was 8900 when measured using GPC (eluent: tetrahydrofuran, HLC-8320GPC EcoSEC; produced by Tosoh Corporation). Shown below are its ¹H-NMR spectrum data that support a structure of regioregular polythiophene represented by formula (1) and show that the regioregularity of the Head-Tail repeating structure is 99% or over.

¹H-NMR (270MHz, CDCl₃, TMS) δ: 3.75 (s, 3H) (at δ 3.88, a peak corresponding to the Head-Head (HH) bond was observed by the intensity of less than 1.0%), 2.69 (br, 2H), 1.6-1.3 (m, 8H), 0.85 (t, 3H, J = 6.5Hz)

### (Example 2)

In a 200ml three-necked flask with a thermometer, 297mg of 3-hexyl-4-methoxythiophene represented by formula (2a), 2.13g of ferric perchlorate and 100ml of acetonitrile were placed. The reaction container was kept at 25°C and stirring was continued for 24 hours to carry out the reaction as shown in chemical reaction equation [IIa]. After the completion of the reaction, reaction liquid was filtrated to obtain a composition having the regioregular polythiophene, a polymer, and a dopant. The obtained polymer was dried and then its electroconductivity was measured to be 1.8 S/cm using a four-probe method.

In a 50ml three-necked flask with a thermometer, the thus obtained polymer, 5ml of hydrazine monohydrate and 5ml of distilled water were placed. The reaction container was kept at 25°C and stirring was continued for 1 hour. After the completion of the reaction, reaction liquid was filtrated and washed with 10ml of distilled water and 10ml of ethanol, then extracted with chloroform. The obtained solution was washed three times with 100ml of distilled water, dried under anhydrous sodium sulfate and concentrated using a rotary evaporator to obtain a polymer, a de-doped regioregular polythiophene. It was found that the obtained polymer in a neutral state is soluble in an organic solvent such as chloroform, methylene chloride, tetrahydrofuran, etc. and has an excellent processability. The number average molecular weight of this regioregular polythiophene was 8700 when measured using the same method as described previously.

Shown below are its ¹H-NMR spectrum data which support the structure of the regioregular polythiophene and show that the regioregularity of the Head-Tail repeating structure is 98.5% or over.

¹H-NMR (270MHz, CDCl₃, TMS) δ: 3.75 (s, 3H) (at δ 3.88, a peak corresponding to the HH bond was observed by the intensity of less than 1.5%), 2.69 (br, 2H), 1.58 (br, 2H), 1.30 (br, 6H), 0.86 (t, 3H, 6.5Hz)

### (Example 3)

In a 50ml three-necked flask with a thermometer and a dropping funnel, 20ml of acetic acid, 10.0g (39.6mmol) of 3-dodecyl thiophene represented by formula (5) shown below and 19.6g (123mmol) of bromide were placed. After the reaction container was heated up to 55°C, stirring was continued for 20 hours to carry out the reaction as shown in chemical reaction equation [III] described below. After the completion of the reaction, 100ml of toluene and 100ml of saturated sodium bicarbonate aqueous solution were added to separate an organic phase from a water phase. The organic phase was washed twice each with 200ml of water, dried under anhydrous sodium sulfate and then concentrated under a reduced pressure, obtaining a crude product. This crude product was purified using a silica gel column chromatography (developing solvent: ethyl acetate and n-hexane), obtaining 2, 3, 5-tribromo-4-dodecyl thiophene (16.0g, 32.7mmol, 83%) represented by formula (6) having properties described below.

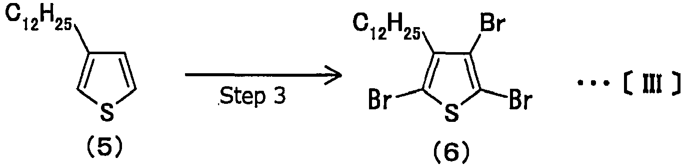

In a 500ml three-necked flask with a thermometer and a dropping funnel, 12.3g (25.1mmol) of 2, 3, 5-tribromo-4-dodecyl thiophene represented by formula (6) and 180ml of tetrahydrofuran were placed. After the reaction container was cooled down to -60°C, 31.4ml (50.2mmol) of n-butyllithium (1.6M) hexane solution was added and stirring was continued for 1 minute to carry out reaction as shown in chemical reaction equation [IV] described below. After the completion of the reaction, 150ml of distilled water and 100ml of toluene were added to separate an organic phase from a water phase. The organic phase was washed twice each with 100ml of water, dried under anhydrous sodium sulfate and then condensed under a reduced pressure, obtaining a crude product. This crude product was purified using a silica gel column chromatography (developing solvent: ethyl acetate and n-hexane, obtaining 3-bromo-4-dodecylthiophene (7.89g, 23.8mmol, 95%) represented by formula (7).

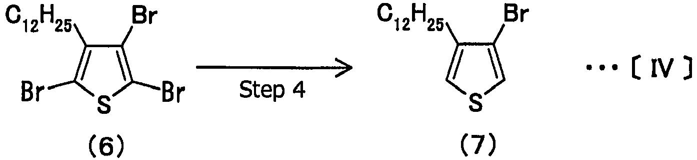

Next, in a 100ml three-necked flask with a thermometer, 7.72g (143mmol) of sodium methoxide, 860mg (6.00mmol) of copper bromide (I), 7.89g (23.8mmol) of 3-bromo-4-dodecylthiophene represented by formula (7) and 15ml of N-methylpyrrolidone were placed. After the reaction container was heated up to 125°C, stirring was continued for 2.5 hours to carry out the reaction as shown in chemical reaction equation [Ib] described below. After the completion of the reaction, 100ml of toluene and 100ml of saturated saline were added to separate an organic phase from a water phase. The organic phase was washed twice each with 100ml of saturated saline, dried under anhydrous sodium sulfate and then concentrated under a reduced pressure, obtaining a crude product. The crude product was purified using a silica gel column chromatography (developing solvent: ethyl acetate and n-hexane, obtaining 3-dodecyl-4-methoxy thiophene (4.28g, 15.2mmol, 64%) represented by formula (2b).

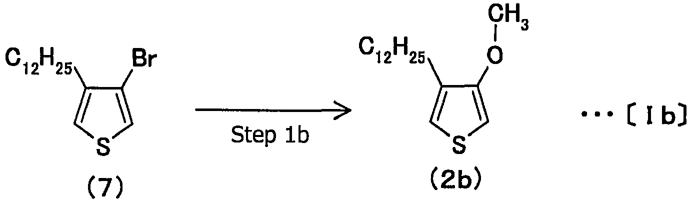

Shown below are its ¹H-NMR spectrum data which support the structure represented by formula (2b).

¹H-NMR (270MHz, CDCl₃, TMS) δ: 6.81 (m, 1H), 6.16 (d, 1H, J = 3.5Hz), 3.81 (s, 3H), 2.46 (t, 2H, J = 7.6Hz), 1.59 (m, 2H), 1.26 (m, 18H), 0.88 (t, 3H, 6.5Hz)

In a 200ml three-necked flask with a thermometer, 423mg of 3-dodecyl-4-methoxythiophene represented by formula (2b), 2.13g of ferric perchlorate and 100ml of acetonitrile were placed. The reaction container was kept at 25°C and stirring was continued for 24 hours to carry out the reaction as shown in chemical reaction equation [IIb]. After the completion of the reaction, the reaction liquid was filtrated to obtain a composition having the regioregular polythiophene, a polymer, and a dopant. The obtained polymer was dried and then its electroconductivity was measured to be 11S/cm using four-probe method.

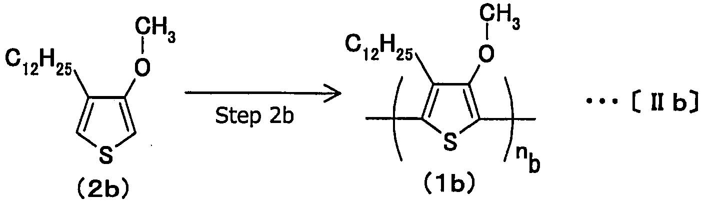

In a 50ml three-necked flask with a thermometer, the thus obtained polymer, 5ml of hydrazine monohydrate and 5ml of distilled water were placed, and the reaction container was kept at 25°C and stirring was continued for 1hour. After completion of the reaction, reaction liquid was filtrated, washed with 10ml of distilled water and 10ml of ethanol, and then extracted with chloroform. The obtained solution was washed three times each with 100ml of distilled water, dried under anhydrous sodium sulfate and concentrated using a rotary evaporator to obtain a polymer, a de-doped regioregular polythiophene. It was found that the obtained neutral state polymer was soluble in an organic solvent such as chloroform, methylene chloride, tetrahydrofuran, etc. and has an excellent processability. The number average molecular weight of this regioregular polythiophene was 9500 when measured using the same method as described previously.

Shown below are its ¹H-NMR spectrum data which support the structure of the regioregular polythiophene and show that the regioregularity of Head-Tail repeating structure is 98.0% or over.

¹H-NMR (270MHz, CDCl₃, TMS) δ: 3.74 (s, 3H), (at δ 3.88, a peak corresponding to the HH bond was observed with the intensity of less than 2.0%), 2.68 (br, 2H), 1.56 (br, 2H), 1.24 (br, 18H), 0.84 (br, 3H)

### (Comparative Example 1)

In a 200ml three-necked flask with a thermometer, 297mg of 3-hexyl-4-methoxythiophene represented by formula (2a), 973mg of ferric chloride and 100ml of acetonitrile were placed. The reaction container was kept at 25°C and stirring was continued for 24 hours. After the completion of the reaction, reaction liquid was filtrated to obtain a polymer. The obtained polymer was dried and then its electroconductivity was measured to be 0.18 S/cm using a four-probe method. In a 50ml three-necked flask with a thermometer, the thus obtained polymer, 5ml of hydrazine monohydrate and 5ml of distilled water were placed, and the reaction container was kept at 25°C and stirring was continued for 1 hour. After the completion of the reaction, the reaction liquid was filtrated, washed with 10ml of distilled water and 10ml of ethanol and then extracted with chloroform. The obtained solution was washed three times each with 100ml of distilled water, dried under anhydrous sodium sulfate and concentrated using a rotary evaporator to obtain a de-doped polymer. The obtained neutral-state polymer was soluble in an organic solvent such as chloroform, methylene chloride, tetrahydrofuran, etc.

Shown below are its ¹H-NMR spectrum data which show an extremely low-regioregularity.

¹H-NMR (270MHz, CDCl₃, TMS) δ: 3.3-4.2 (br, 3H) (at δ 3.90, a peak corresponding to the HH bond was observed by the intensity of about 25% and a peak corresponding to the HT bond (at δ 3.74) was observed by the intensity of about 75%), 2.68 (br, 2H), 1.20-1.70 (br, 8H), 0.87 (br, 3H)

### (Comparative Example 2)

In a 200ml three-necked flask with a thermometer, 297mg of 3-hexyl-4-methoxythiophene represented by formula (2a), 2220mg of six hydrate cupric perchlorate and 100ml of acetonitrile were placed. The reaction container was kept at 25°C and stirring was continued for 24 hours. After the completion of the reaction, the reaction liquid was filtrated but no extract and therefore no polymer was observed.

### Industrial Applicability

The regioregular polythiophene of the present invention can be used as a raw material for conductive material. Further, the conductive material containing the regioregular polythiophene is useful as conductive organic material for an electrodes, sensors, primary cells, secondary cells, solid electrolytic capacitors, antistatic agents, etc.

## Claims

1. A regioregular polythiophene represented by the formula (1), 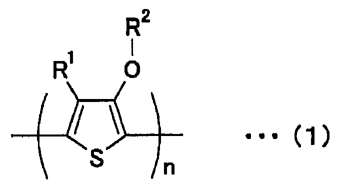 (in formula (1): R¹- represents a 4-substituent comprising an alkyl having 1 to 20 carbon atoms; R²-O- represents a 3-substituent comprising an alkyl having 1 to 20 carbon atoms; and n represents such a numeral value that the number average molecular weight of the regioregular polythiophene becomes 200 to 1,000,000),
wherein the regioregularity of the Head-Tail repeating structure is at least 98%.

2. A raw material composition of a conductive material, which comprises a polythiophene represented by the formula (1), and a dopant, 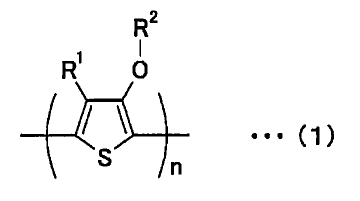 (in formula (1): R¹- represents a 4-substituent comprising an alkyl having 1 to 20 carbon atoms; R²-O- represents a 3-substituent comprising an alkyl having 1 to 20 carbon atoms; and n represents such a numeral value that the number average molecular weight of the regioregular polythiophene becomes 200 to 1,000,000),
wherein the regioregularity of the Head-Tail repeating structure is at least 98%.

3. A process for producing a polythiophene, comprising a step of:
polymerizing a thiophene compound monomer represented by formula (2), 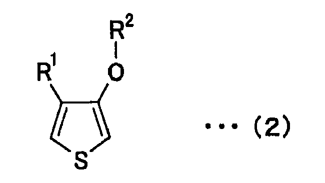 (in formula (2): R¹- represents a 4-substituent comprising an alkyl having 1 to 20 carbon atoms; R²-O- represents a 3-substituent comprising an alkyl having 1 to 20 carbon atoms), using an electrolytic polymerization method or a chemical oxydative polymerization method in the presence of ferric perchlorate,
to induce to the polythiophene represented by formula (1), 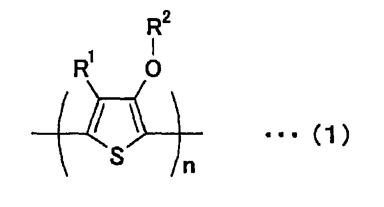 (in formula (1): R¹- and R²-O- are the same as described in the formula (2) shown above, and n represents such a numeral value that the number average molecular weight of the regioregular polythiophene becomes 200 to 1,000,000)
wherein the regioregularity of the Head-Tail repeating structure is at least 98%.

4. A thiophene compound monomer represented by formula (3) 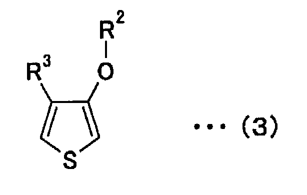 (in formula (3): R³- is a 4-substituent comprising an alkyl having 6 to 20 carbon atoms; R²-O- represents a 3-substituent comprising an alkyl having 1 to 20 carbon atoms).
